# EUROPÄISCHE PATENTSCHRIFT

(11) **EP 1 180 994 B1**
(45) Veröffentlichungstag und Bekanntmachung des Hinweises auf die Patenterteilung: **07.05.2003**
(21) Anmeldenummer: 00936840.8
(22) Anmeldetag: 31.05.2000
(51) Int. Cl.: A61F 13/02, B65H 35/02, B65H 20/32, B65H 23/32, B65H 39/16

(54) **VERFAHREN UND VORRICHTUNG ZUM HERSTELLEN EINES PRODUKTES AUS STREIFENBAND, INSBESONDERE EINES MEDIZINISCHEN UND/ODER WIRKSTOFFHALTIGEN PRODUKTES SOWIE BEFÜLLBAREN BEHÄLTERN ODER SIEGELRANDBEUTELN**
METHOD AND DEVICE FOR PRODUCING A PRODUCT MADE OF STRIP TAPE, ESPECIALLY A MEDICAL PRODUCT AND/OR A PRODUCT CONTAINING ACTIVE SUBSTANCES AS WELL AS FILLABLE RECEPTACLES OR POUCHES WHOSE EDGES CAN BE SEALED
PROCEDE ET DISPOSITIF POUR PREPARER UN PRODUIT A BASE DE BANDE STRIEE, NOTAMMENT UN PRODUIT A USAGE MEDICAL ET/OU CONTENANT UN PRINCIPE ACTIF, AINSI QUE DES RECIPIENTS OU DES SACHETS A BORDS SCELLABLES, REMPLISSABLES

(30) Priorität: 02.06.1999 DE 19925339
(43) Veröffentlichungstag der Anmeldung: 27.02.2002
(73) Patentinhaber: LTS Lohmann Therapie-Systeme AG, 56626 Andernach (DE)
(72) Erfinder: NEULAND, Detlev, D-56645 Nickenich (DE); HOFFMANN, Hans-Rainer, D-56566 Neuwied (DE); SCHÄFER, Wolfgang, D-53757 St. Augustin (DE)
(74) Vertreter: Flaccus, Rolf-Dieter, Dr.
(86) Internationale Anmeldenummer: EP0004970
(87) Internationale Veröffentlichungsnummer: WO00074618

(56) Entgegenhaltungen:
- EP-A- 0 822 155
- EP-A- 0 848 937
- DE-A- 2 709 211
- DE-C- 211 962
- US-A- 2 293 178
- US-A- 2 332 544
- US-A- 3 756 527
- US-A- 5 172 621
- US-A- 5 571 361
- PATENT ABSTRACTS OF JAPAN vol. 010, no. 375 (M-545), 13. Dezember 1986 (1986-12-13) -& JP 61 166467 A (MANSURII SHOKAI:KK), 28. Juli 1986 (1986-07-28)
- PATENT ABSTRACTS OF JAPAN vol. 012, no. 208 (M-709), 15. Juni 1988 (1988-06-15) -& JP 63 012567 A (TOPPAN PRINTING CO LTD), 19. Januar 1988 (1988-01-19)

## Beschreibung

Die Erfindung betrifft ein Verfahren zum Herstellen eines Produktes aus Streifenband, insbesondere eines medizinischen und/oder wirkstoffhaltigen Produktes wie beispielsweise dermales oder transdermales Pflaster bzw. eine andere Darreichungform, beispielsweise zur oralen Applikation, sowie befüllbaren Behältern oder Siegelrandbeuteln, wobei als Ausgangsmaterial eine breite wirkstoffhaltige Materialbahn, beispielsweise aus folienförmigen Materialien, und insbesondere aus wirkstoffhaltiger Folie oder folienförmigem Wirkstoff, verwendet wird, umfassend mindestens zwei der Arbeitsschnitte:
- Zerteilen der breiten Materialbahn in einzelne schmale Streifen und gegebenenfalls Aufwickeln der Streifen zu einzelnen Spulen, bzw. gemeinsames Drehen der Streifen,
- Abwickeln bedarfsweise einzelner Spulen bzw. Spulenpaare und Zusammenfügen jeweils wenigstens zweier Streifen zu einer Streifenbahn, bzw. Abwickeln der aus Einzelstreifen aufgerollten Streifenbahn,
- Verarbeiten der Materialbahn zu einem aus Streifenband gebildeten Produkt,
- Durchführung finaler Arbeitsschritte wie z.B. Konfektionieren des Endproduktes, Ausbilden der endgültigen Applikationsform, Kaschieren eines Trägermaterials, Vereinzeln, Verpacken, etc.

Zum Herstellen der vorgenannten Produkte wird Streifenband, bestehend aus mehreren einzelnen Streifen, verwendet.

Dieses Band wird hergestellt, indem eine Materialrolle des Ausgangsmaterials mit der Breite, die sich aus der Anzahl der Streifen, multipliziert mit der Streifenbreite plus Randbeschnitt, ergibt, zu einzelnen Streifen zertrennt wird.
Üblicherweise werden die erhaltenen Streifen auf Spulen gewickelt und bedarfsweise in erforderlicher Anzahl gemeinsam abgewickelt und zu mindestens zweischichtigen Materialbahnen zusammengefügt.
Die Herstellung der Bevorratungsspulen ist sehr arbeitsaufwendig, ebenso das Auf- und Abwickeln der einzelnen Streifen.
Dabei kann es vorkommen, daß das Material, bei dem es sich um ein wirkstoffhaltiges Material oder ein Arzneimittel handeln kann, unzulässig gestreckt wird, wobei sich die unvermeidliche Dehnung des Materials nachteilig auf die Dosiergenauigkeit des Arzneimittels auswirkt.

US-A-2 332 544 beschreibt ein Verfahren gemäß des Oberbegriffs des Anspruchs 1 und eine Vorrichtung gemäß des Oberbegriffs des Anspruchs 5.

Der Erfindung liegt die Aufgabe zugrunde, ein Verfahren und eine zu seiner Durchführung geeignete Vorrichtung anzugeben, welche die vorgenannten Nachteile, Schwierigkeiten und technischen Grenzen überwindet und insbesondere die Herstellung eines Streifenbandproduktes, besonders bevorzugt eines wirkstoffhaltigen Produktes mit verringertem Aufwand an Arbeit und Kosten ohne nachteilige Dehnung des Materials ermöglicht.

Zur Lösung der Aufgabe wird bei einem Verfahren der eingangs genannten Art mit der Erfindung vorgeschlagen,
- daß eine Materialrolle mit einer breiten Materialbahn des Ausgangsmaterials lose drehbar auf einen Aufnahmedorn aufgesteckt wird,
- daß die Materialbahn in gesamter Breite mittels einer Vakuumwalze, oder einer Vorrichtung aus zwei Walzen, die das Material schonend von der Materialrolle abwickelt,
wobei eine der Walzen gleichzeitig als Gegenhalt der Schneidmesser dient, ohne Einwirkung einer Zugspannung von der Materialrolle abgezogen und dabei im Unterdruckbereich der Vakuumwalze durch Abrollen einer Vielfach-Rundmesserwalze in einzelne Streifen zerteilt,
- daß jeder Streifen am Ende des Unterdruckbereiches der Vakuumwalze oder des Walzenpaares von dieser oder diesem abgezogen und in einen Aufnahmekanal eingeführt und darin mittels Unterdruck kontinuierlich gefördert wird,
- daß dabei jeder Streifen auf seinem Wege zum Kanal oder durch den Kanal um ca. 90° gedreht wird, und
- daß am Ende der Kanäle die Streifen jeweils wenigstens zu zweit übereinander geführt und danach in einer vorzugsweise offenen Rinne zur weiteren Verarbeitung unter Komplettierung zur Endform des Produktes in eine Konfektionierungsmaschine weitertransportiert werden und darin fertig konfektioniert werden.

Eine Ausgestaltung des Verfahrens sieht vor, daß zum Abziehen der Materialbahn und Verteilen in Streifen im Zusammmenwirken mit der Vielfach-Rundmesserwalze anstelle einer Vakuumwalze eine glatte Abziehwalze verwendet wird, welche in einem Umschlingungsbereich der Materialbahn mit je einer Andrückrolle und einer Führungsrolle zusammenwirkt.

Eine Vorrichtung zum erfindungsgemäßen Herstellen eines Produktes aus Streifenband, insbesondere eines medizinischen und/oder wirkstoffhaitigen Produktes wie beispielsweise dermales oder transdermales Pflaster bzw. eine andere Darreichungsform, beispielsweise zur oralen Applikation, sowie befüllbaren Behältern oder Siegelrandbeuteln, insbesondere zur Durchführung des Verfahrens nach der Erfindung, ist gekennzeichnet durch die Merkmale:
- daß als Mittel zum Abziehen und Verteilen der Materialbahn eine Vakuumwalze vorgesehen ist, an deren Umlaufbereich, vom Unterdruck gehalten, die Materialbahn im Zusammenwirken mit der Vielfach-Messerwalze in einzelne Streifen zerteilbar ist,
- daß jedem Streifen ein Vakuum-Förderkanal zugeordnet werden kann, der so ausgebildet ist, daß jeder Streifen auf seinem Weg zum Kanal oder durch den Kanal um ca. 90° gedreht wird, und
- daß die Enden aller Aufnahmekanäle 12 an einer Stelle zu einer Einheit zusammengeführt und dort Führungs- und Transportmittel zum Weitertransport an eine Konfektionierungsmaschine vorgesehen sind.

Eine Ausgestaltung der Vorrichtung nach der Erfindung sieht vor, daß als Mittel zum Abziehen der Materialbahn und zum Transport beim Zerteilen in Streifen im Zusammenwirken mit einer Vielfach-Rundmesserwalze anstelle einer Vakuumwalze eine glatte Abziehwalze vorgesehen ist, welche in einem Umschlingungsbereich der Materialbahn mit je einer Andrückrolle und einer Führungsrolle zusammenwirkungsfähig angeordnet und ausgebildet ist.

Mit dem erfindungsgemäßen Verfahren und der zu seiner Durchführung vorgesehenen Vorrichtung werden die vorgenannten Nachteile, Schwierigkeiten und technischen Grenzen des Standes der Technik überwunden und insbesondere eine Herstellung von medizinischen und/oder wirkstoffhaltigen Produkten mit verringertem Aufwand an Arbeit und Kosten ohne nachteilige Dehnung des Ausgangsmaterials ermöglicht.

Weitere Einzelheiten, Merkmale und Vorteile der Erfindung ergeben sich aus der nachstehenden Erläuterung eines in den Zeichnungen schematisch dargestellten Ausführungsbeispieles.
Es zeigen :
- FIG.1:: Eine Ansicht der Vorrichtung von der Seite;
- FIG.2:: Die Vorrichtung gemäß FIG.1 in Draufsicht;
- FIG.3:: Eine teilweise Ansicht der Vorrichtung mit an deren Ende angeordneten Vakuumtransportkanälen;
- FIG.4:: Eine Ansicht der Vorrichtung, bei welcher anstelle einer Vakuumwalze eine glatte Abziehwalze vorgesehen ist.

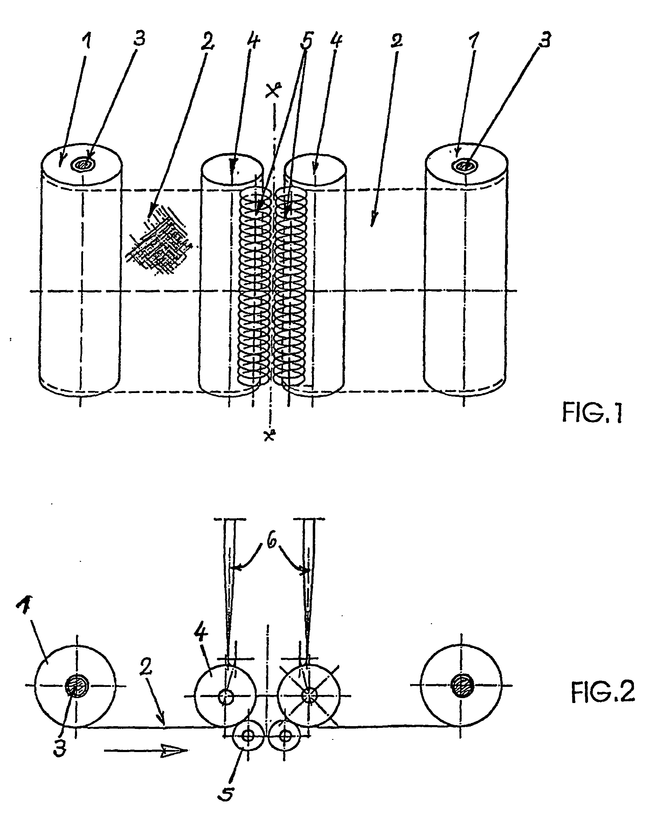

Der in FIG.1 dargestellte Teil der Vorrichtung zeigt in einer Doppelanordnung zu beiden Seiten einer Symmetrieebene x-x Materialrollen 1 mit einer Materialbahn 2 des Ausgangsmaterials, aufgesteckt auf Aufnahmedorne 3, sowie eine Doppelanordnung von Vakuumwalzen 4 und auf diesen bzw. der Materialbahn 2 abrollende Rundmesserwalzen 5 mit einer Vielzahl parallel angeordneter Rundmesser.
Die gleiche Vorrichtung ist in FIG.2 in Draufsicht gezeigt mit Materialwalzen 1, Aufsteckdorn 3, abrollbarer Materialbahn 2, Vakuumwalzen 4, Rundmesserwalzen 5 und von diesen am Ende des Vakuumbereichs ablaufenden, aus der Materialbahn 2 geschnittenen Materialstreifen 6.

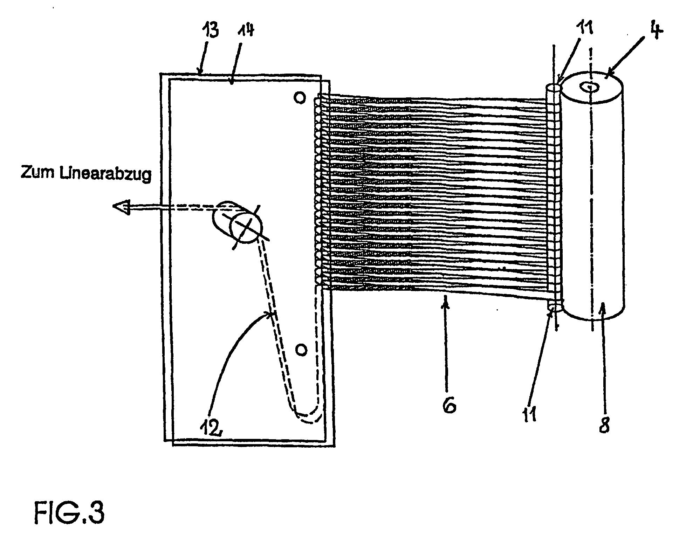

FIG.3 zeigt teilweise in Seitenansicht und teilweise in Draufsicht eine Vakuumwalze 4. Es kann sich jedoch auch um eine Abziehwalze 8 mit Unterstützung durch eine Führungsrolle 11 handeln, von der mit einer nicht dargestellten Rundmesserwalze geschnittene Streifen 6 in Breite der ursprünglichen Materialbahn 2 abtransportiert werden. Zum Linearabzug einer Vielzahl von Streifen 6 sind Vakuumförderkanäle 12 vorgesehen und beispielsweise zwischen jeweils zwei Abdeckplatten 13 und 14 ausgebildet.

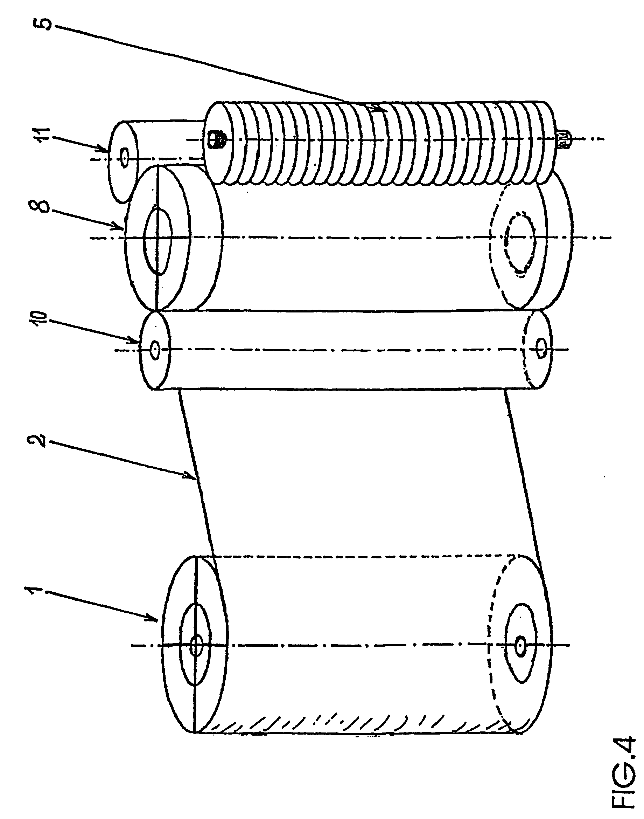

Schließlich zeigt FIG.4 eine alternative Ausbildung der Vorrichtung, bei welcher als Mittel zum Abziehen der Materialbahn 2 und zum Transport beim Verteilen der Materialbahn in Streifen 6 im Zusammenwirken mit einer Vielfach-Rundmesserwalze 5 anstelle einer Vakuumwalze eine glatte Abziehwalze 8 vorgesehen ist, welche in einem Umschlingungsbereich 9 die Materialbahn 2 mit je einer Andrückrolle 10 und einer Führungsrolle 11 zusammenwirkungsfähig angeordnet und ausgebildet ist.

Das Verfahren und die Vorrichtung sind unkompliziert, verringern den bisher erforderlichen Aufwand an Arbeit und Kosten und ermöglichen die Herstellung aus Streifenband von medizinischen und/oder wirkstoffhaltigen Produkten verschiedener Ausbildung oder befüllbaren Behältern oder Siegelrandbeuteln unter Vermeidung einer nachteiligen Dehnung des Materials, und insbesondere unter Verwendung von Ausgangsmaterial in Form von folienförmigem Material, bevorzugt einer folienförmigen wirkstoffhaltigen Darreichungsform.

Insofern löst die Erfindung in optimaler Weise die eingangs gestellte Aufgabe.

## Patentansprüche

1. Verfahren zum Herstellen eines Produktes aus Streifenband, insbesondere eines medizinischen und/oder wirkstoffhaltigen Produktes wie beispielsweise dermales oder transdermales Pflaster bzw. eine andere Darreichungsform, beispielsweise zur oralen Applikation, sowie befüllbaren Behältern oder Siegelrandbeuteln, wobei
eine Materialrolle (1) mit einer breiten Materialbahn (2) lose drehbar auf einen Aufnahmedorn (3) aufgesteckt wird,
- daß die Materialbahn (2) in gesamter Breite mittels einer Vakuumwalze (4) ohne Einwirkung einer Zugspannung von der Materialrolle (1) abgezogen und dabei im Unterdruckbereich der Vakuumwalze (4) durch Abrollen einer Vielfach-Rundmesserwalze (5) in einzelne Streifen (6) zerteilt,
- daß jeder Streifen (6) am Ende des Unterdruckbereiches der Vakuumwalze (4) von dieser abgezogen wird **dadurch gekennzeichnet,**
- **daß** jeder Streifen in einen Aufnahmekanal (7) eingeführt und darin mittels Unterdruck kontinuierlich gefördert wird,
- **daß** dabei jeder Streifen (6) auf seinem Wege zum Kanal oder durch den Kanal (7) um ca. 90° gedreht wird, und
- **daß** am Ende der Kanäle (7) die Streifen (6) jeweils wenigstens zu zweit übereinander geführt und danach in einer vorzugsweise offenen Rinne zur weiteren Verarbeitung unter Komplettierung zum fertigen medizinischen und/oder wirkstoffhaltigen Produkt oder befüllbaren Behältern oder Siegelrandbeuteln in eine Konfektionierungsmaschine weitertransportiert und darin fertig konfektioniert werden.

2. Verfahren nach Anspruch 1, **dadurch gekennzeichnet, daß** als breite Materialbahnen folienförmige Materialien verwendet werden.

3. Verfahren nach Anspruch 1, **dadurch gekennzeichnet, daß** als breite Materialbahnen wirkstoffhaltige folienförmige Darreichungsformen verwendet werden.

4. Verfahren nach Anspruch 1, **dadurch gekennzeichnet, daß** zum Abziehen der Materialbahn (2) und Zerteilen in Streifen (6) im Zusammenwirken mit der Vielfach-Rundmesserwalze (5) anstelle einer Vakuumwalze eine glatte Abziehwalze (8) verwendet wird, welche in einem Umschlingungsbereich der Materialbahn (2) mit je einer Andrückrolle (10) und einer Führungsrolle (11) zusammenwirkt.

5. Vorrichtung zum Herstellen eines Produktes aus Streifenband, insbesondere eines medizinischen und/oder wirkstoffhaltigen Produktes wie beispielsweise dermales oder transdermales Pflaster bzw. eine andere Darreichungsform, beispielsweise zur oralen Applikation, sowie befüllbaren Behältern oder Siegelrandbeuteln, insbesondere zur Durchführung des Verfahrens nach der Erfindung, wobei
als Mittel zum Abziehen und Verteilen der Materialbahn (2) eine Vakuumwalze (4) vorgesehen ist, an deren Umlaufbereich, vom Unterdruck gehalten, die Materialbahn (2) im Zusammenwirken mit der Vielfach-Messerwalze (5) in einzelne Streifen (6) zerteilbar ist,
**dadurch gekennzeichnet,**
- **daß** jedem Streifen (6) ein Vakuum-Förderkanal (12) zugeordnet und so ausgebildet ist, daß jeder Streifen (6) auf seinem Weg zum Kanal oder durch den Kanal (12) um ca. 90° gedreht wird, und
- **daß** Enden aller Aufnahmekanäle (12) an einer Stelle zu einer Einheit zusammengeführt und dort Führungs- und Transportmittel zum Weitertransport an eine Konfektionierungsmaschine vorgesehen sind.

6. Vorrichtung nach Anspruch 5, **dadurch gekennzeichnet, daß** als Mittel zum Abziehen der Materialbahn (2) und zum Transport beim Zerteilen in Streifen (6) im Zusammenwirken mit einer Vielfach-Rundmesserwalze (5) anstelle einer Vakuumwalze eine glatte Abziehwalze (8) vorgesehen ist, welche in einem Umschlingungsbereich der Materialbahn (2) mit je einer Andrückrolle (10) und einer Führungsrolle (11) zusammenwirkungsfähig angeordnet und ausgebildet ist.

## Claims

1. Process for manufacturing a product from strip tape, in particular a medicinal and/or active substance-containing product such as, for example, a dermal or transdermal patch or another administration form, for example for oral application, as well as fillable containers or sealed-margin bags, wherein
- a roll of material (1) with a broad web of material (2) is mounted, loosely rotatable, on a take-up mandrel (3),
- a web of material (2) is drawn, in its entire width and without subjecting the material to tensile stress, from the roll of material (1) by means of a vacuum roll (4), and is separated in the process into individual strips (6) in the negative pressure zone of the vacuum roll (4) by rolling a multiple circular knife roll (5),
- at the end of the negative pressure zone of the vacuum roll (4), each strip (6) is drawn from the said vacuum roll (4), **characterized in that** each strip is introduced in a take-up channel (7) and continuously conveyed therein my means of negative pressure,
- in the process, each strip (6) is turned on its way to the channel or through the channel (7) by about 90°, and
- at the end of the channels (7) the strips (6) are led one upon the other, at least two at a time, and thereafter conveyed further in a, preferably open, groove for further processing under completion to form the finished medicinal and/or active substance-containing product or fillable containers or sealed-margin bags in a conversion equipment where the products are finished.

2. Process according to Claim 1, **characterized in that** as broad webs of material, sheet-like materials are used.

3. Process according to Claim 1, **characterized in that** as broad webs of material, active substance-containing sheet-like administration forms are used.

4. Process according to Claim 1, **characterized in that** for drawing the web of material (2) and separating the same into strips (6) in cooperation with a multiple circular knife roll (5) there is used instead of a vacuum roll, a smooth stripping roll (8) which in a zone in which the material web (2) travels around the roll cooperates with a pressure roll (10) and a guide roll (11).

5. Device for manufacturing a product from strip tape, especially a medicinal and/or active substance-containing product such as, for instance, a dermal or transdermal patch or another administration form, for example for oral application, as well as fillable containers or sealed-margin bags, especially for carrying out the process according to the invention, whereby
- as a means for drawing off and separating the web of material (2) a vacuum roll (4) is provided, in whose web-travelling zone the web of material (2), held by the negative pressure, can be severed into individual strips (6) in cooperation with the multiple circular knife roll (5),
**characterized in that** to each strip (6) there is associated a vacuum conveyor channel (12) which is designed such that each strip (6) is turned on its way to the channel or through the channel (12) by about 90°, and
- the ends of all the take-up channels (12) are brought together at one site to form a unit and that at that site there are provided guide means and transport means for the further transport to a conversion equipment.

6. Device according to Claim 5, **characterized in that** as means for drawing the web of material (2) and for transport during the separation into strips (6) in cooperation with a multiple circular knife roll (5), there is provided instead of a vacuum roll, a smooth stripping roll (8) arranged in a zone where the web of material (2) travels around the roll and adapted to cooperate with a pressure roll (10) and a guide roll (11).

## Revendications

1. Dispositif pour fabriquer un produit à base de bande striée, notamment un produit à usage médical et/ou contenant un principe actif, par exemple un emplâtre dermique ou transdermique, ou bien une autre forme d'administration, par exemple pour une administration orale, ainsi que des récipients ou des sachets à bords scellables remplissables, dans lequel
- un rouleau de matériau (1) avec une large bande de matériau initial (2) tournant facilement est placé sur un mandrin d'admission (3),
- la bande de matériau (2) dans sa largeur totale par l'intermédiaire d'un cylindre sous vide (4) est retirée du rouleau de matériau (1) sans influence d'une contrainte de tension, et est ainsi découpée en stries individuelles (6) dans la zone de sous-pression du cylindre sous vide (4) par le déroulage d'un arbre porte-lames circulaire multiple (5),
- chaque strie (6) est retirée à la fin de la zone de sous-pression du cylindre sous vide (4) de celui-ci, **caractérisé en ce que**
- chaque strie est introduite dans un canal d'admission (7), et est déplacée en continu à l'intérieur de celui-ci à l'aide de la sous-pression,
- chaque strie (6) sur son trajet vers le canal ou à travers le canal (7) est tournée d'environ 90°, et
- à la fin des canaux (7), les stries (6) sont conduites à chaque fois au moins par deux l'une sur l'autre, puis transportées dans une gouttière de préférence ouverte pour un traitement supplémentaire afin de compléter la réalisation de la forme finie du produit à usage médical et/ou contenant un principe actif, ou des récipients ou des sachets à bords scellables remplissables dans une machine de confection, et sont confectionnées à l'intérieur de celle-ci.

2. Procédé selon la revendication 1, **caractérisé en ce que,** comme feuilles larges de matériaux, des matériaux en forme de feuille sont utilisés.

3. Procédé selon la revendication 1, **caractérisé en ce que,** comme bandes larges de matériaux, des formes d'administration en forme de feuille contenant des principes actifs sont utilisées.

4. Procédé selon la revendication 1, **caractérisé en ce que,** pour retirer la bande de matériau (2) et la découper en stries (6) en action conjointe avec l'arbre porte-lames circulaire multiple (5) à la place d'un cylindre sous vide est utilisé un cylindre de tirage lisse (8), lequel dans une zone d'enroulage de la bande de matériau (2) agit conjointement à chaque fois avec un galet presseur (10) et un galet de guidage (11).

5. Dispositif pour fabriquer un produit à base de bande striée, notamment un produit à usage médical et/ou contenant un principe actif, par exemple un emplâtre dermique ou transdermique, ou bien une autre forme d'administration, par exemple pour une administration orale, ainsi que des récipients ou des sachets à bords scellables remplissables, en particulier pour la réalisation du procédé selon l'invention, dans lequel
- comme moyen de retirer et de découper la bande de matériau (2), il est prévu un cylindre sous vide (4), où, au niveau de sa zone de rotation, maintenue par la sous-pression, la bande de matériau (2) peut être découpée en stries individuelles (6) conjointement avec l'arbre porte-lames multiple (5),
- **caractérisé en ce qu'à** chaque strie (6) est attribué un canal d'extraction sous vide (12), et qui est conçu de telle manière que chaque strie (6) est tournée sur son trajet vers le canal ou à travers le canal (12) d'environ 90°, et
- les terminaisons de tous les canaux d'admission (12) sont réunies à un endroit, et, à cet emplacement, sont prévus des moyens de guidage et de transport vers une machine de confection.

6. Dispositif selon la revendication 5, **caractérisé en ce que,** comme moyen de retirer la bande de matériau (2) et de la transporter lors de sa découpe en stries (6) en action conjointe avec l'arbre porte-lames circulaire multiple (5) à la place d'un cylindre sous vide est utilisé un cylindre de tirage lisse (8), lequel dans une zone d'enroulage de la bande de matériau (2) est placé et conçu pour agir conjointement à chaque fois avec un galet presseur (10) et un galet de guidage (11).
